(19) Europäisches Patentamt
European Patent Office
Office européen des brevets

(11) **EP 4 623 300 B1**

(12) **EUROPEAN PATENT SPECIFICATION**

(45) Date of publication and mention
of the grant of the patent:
**29.04.2026 Bulletin 2026/18**

(21) Application number: **23806319.2**

(22) Date of filing: **17.11.2023**

(51) International Patent Classification (IPC):
***G01N 33/543*** (2006.01)   ***G01N 33/58*** (2006.01)

(52) Cooperative Patent Classification (CPC):
**G01N 33/54373; G01N 33/54346; G01N 33/587;**
G01N 2333/42; G01N 2400/00

(86) International application number:
**PCT/EP2023/082173**

(87) International publication number:
**WO 2024/110324 (30.05.2024 Gazette 2024/22)**

(54) **MBL DETECTION ASSAY**

MBL-NACHWEISTEST

DOSAGE DE DÉTECTION DE MBL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **25.11.2022 EP 22209662**

(43) Date of publication of application:
**01.10.2025 Bulletin 2025/40**

(73) Proprietor: **VITO NV**
**2400 Mol (BE)**

(72) Inventors:
 • **PANCARO, Alessia**
  2400 Mol (BE)
 • **NELISSEN, Inge**
  2400 Mol (BE)
 • **SZYMONIK, Michal**
  2400 Mol (BE)

(74) Representative: **Arnold & Siedsma**
**Bezuidenhoutseweg 57**
**2594 AC The Hague (NL)**

(56) References cited:
 • **WANG Y ET AL: "Multivalent interaction-based carbohydrate biosensors for signal amplification", BIOSENSORS AND BIOELECTRONICS, ELSEVIER SCIENCE LTD, UK, AMSTERDAM , NL, vol. 26, no. 3, 15 November 2010 (2010-11-15), pages 996 - 1001, XP027393248, ISSN: 0956-5663, [retrieved on 20100819]**
 • **PANCARO ALESSIA ET AL: "The polymeric glyco-linker controls the signal outputs for plasmonic gold nanorod biosensors due to biocorona formation", vol. 13, no. 24, 24 June 2021 (2021-06-24), United Kingdom, pages 10837 - 10848, XP055934470, ISSN: 2040-3364, Retrieved from the Internet <URL:https://pubs.rsc.org/en/content/articlepdf/2021/nr/d1nr01548f> DOI: 10.1039/D1NR01548F**
 • **YOUNG JU SON ET AL: "Multifunctional Nanorods Serving as Nanobridges To Modulate T Cell-Mediated Immunity", ACS NANO, vol. 7, no. 11, 26 November 2013 (2013-11-26), US, pages 9771 - 9779, XP055596647, ISSN: 1936-0851, DOI: 10.1021/nn403275p**

**Description**

FIELD OF THE INVENTION

The present invention is directed to an assay for the detection of Mannose-binding lectin (MBL) using localized surface plasmon resonance (LSPR) of gold nanorods (GNRs) coated with lectin-binding glyco-polymers.

BACKGROUND TO THE INVENTION

Mannose-binding lectin (MBL) is a collagen-like serum protein and a major component of the innate immune system. The main function of MBL is the activation of the lectin pathway of the complement system and the subsequent inflammatory mechanisms.

The incidence and outcome of many human diseases, such as myocardial infarction, and gastrointestinal, kidney, and cerebral ischemia, are associated and influenced by the activity and serum concentrations of MBL in the body fluids. Increased MBL serum concentration and activity have been associated with inflammatory autoimmune diseases, transplant rejection, diabetic nephropathy, mycobacteria infection, leishmania, and primary biliary cirrhosis. On the other hand, low MBL plasmatic levels, mainly due to genetic influences, are associated with a higher risk, severity, and frequency of infections, and worse outcomes during severe systemic infections, such as sepsis. Low serum MBL levels are also reported to be correlated with recurrent spontaneous abortions and a negative impact on pregnancy outcome in women with a history of unexplained recurrent spontaneous abortion.

Monitoring MBL serum levels could be beneficial for many diagnostic and therapeutic applications and serological tests are mostly used due to the non-invasive nature and convenience. To monitor MBL levels, currently tests based on the enzyme-linked immunosorbent assay (ELISA) are used. However, this technique has several limitations such as: long response time typically 3-4.5 hours, that are not compatible with diagnosis that requires fast delivery; numerous washing steps; narrow dynamic range that requires testing samples at different dilutions to fall within the linear range; short signal stability requiring reading within minutes of adding the stop solution; weak protein-protein (or antibody-protein) interactions may not be detected due to the numerous washing steps in ELISA assays.

Another technique employed to measure MBL has been based on a surface plasma resonance (SPR) assay using a chip surface functionalized with a pattern of sugar moieties (Erol G, Schmidt PP, Pancaro A, et al. New nanostructures inhibiting human mannose binding lectin identified by a novel surface plasmon resonance assay. Sensors Actuators B Chem. 2022;360(March):131661. doi:10.1016/j.snb.2022.131661). Also PANCARO ALESSIA ET AL: "The polymeric glyco-linker controls the signal outputs for plasmonic gold nanorod biosensors due to biocorona formation", NANOSCALE, vol. 13, no. 24 24 June 2021 (2021-06-24), pages 10837-10848, discloses a localized surface plasmon resonance (LSPR) lectin binding assay comprising the use of the galactosamine-functionalized GNRs (provided in the form of a stable colloidal monodisperse suspension). However, SPR is not commonly used in clinical practice due to meticulous experimental design with long calibration process. SPR is heavily affected by environmental noise/external influences such as temperature, medium changes, as well as nonspecific binding. SPR consists of several sequential steps: (i) immobilization of the ligand to the sensor surface, (ii) blocking of the remaining binding sites, (iii) analyte injection and binding to the ligand to form a complex (association phase), (iv) dissociation of the complex for further analysis, also by employing stringent washing of several different pH solutions or different ionic strengths, and (v) elution of captured proteins to reuse the sensor that can be challenging as the ligand-analyte complex usually has a rather high affinity.

Moreover, it is necessary to have expensive scientific cameras, gold chips and equipment. As a consequence SPR can only measure molecular interactions in well-defined buffers and due to non-specific binding and the microfluidic system, is not possible to measure MBL directly in undiluted human serum.

It has accordingly been an object of the present invention to develop an alternative MBL detection assay for direct measurement in biological fluids, that consists of a fast, one-step, wash-free process with no dilutions (or only a low scale dilution such as a single dilution of between 1:2 up to 1:5) are required.

SUMMARY OF THE INVENTION

The invention is defined in the claims. The present invention is based on the development of mannose-functionalized gold nanorods that can be used directly in bodily fluid samples such as blood and serum samples for MBL detection.

It accordingly provides mannose-functionalized gold nanorods (GNRs) wherein the mannose is linked to the gold nanorods by a thiol-polyethylene glycol (PEG)-based polymer, characterized in comprising an alkane linker between the PEG and the thiol group, and wherein the thiol group is bound to the gold surface of the gold nanorods. As will become apparent from the examples hereinafter, it has been observed that the presence of the thiol-polyethylene glycol (PEG)-based polymer prevents aggregation of the GNRs in said environment of bodily fluids and accordingly enables a direct MBL measurement, when applied with a surface coverage of at least 3 molecules per nm$^2$ to the surface of the gold nanorods.

In as far such mannose-functionalized gold nanorods (GNRs) are known from Wang Y. et al., Biosensors and Bioelectronics, Vol. 26, No. 3, 15 November 2010 or from Young Ju Son et al., ACS Nano, Vol; 7, No. 11, 26 November 2013; in neither of said references these GNRs have been disclosed in the framework of MBL detection, let alone the surface coverage to resolve the nanoparticle aggregation induced instability which compromises the assay.

In one aspect the according to the invention mannose-functionalized GNRs comprise a thiol-polyethylene glycol (PEG) based polymer linker represented by the following formula (I)

$$-S-(CH2)n-(OCH2CH2)m-O-\qquad(I)$$

wherein n represents an integer from 2 to 20; in particular from 6 to 16, more in particular from 8 to 14, even more in particular from 9 to 13, and m represents an integer from 2 to 15; in particular from 3 to 14, more in particular from 4 to 12, even more in particular from 4 to 8.

The mannose-functionalized GNRs preferably consist of long GNRs, these are particularly suitable in having a strong extinction in the near-infrared range, typically from about 600 nm to about 1300 nm, and in having a length which is larger than the width of the rods, usually expressed as the aspect ratio, i.e. the length of the GNRs over the width of the GNRs. In an embodiment the GNRs are long GNRs with an aspect ratio of at least 2. Long GNRs with strong extinction in the near-infrared range enable simple measurement on a UV/visible/NIR spectrometer. In a particular embodiment the long GNRs have an aspect ratio of about and between 3.0 to 4.5; more in particular of about and between 3.4 to 4.0; even more in particular of about and between 3.6 to 3.8. Geometric properties of the GNRs are also reflected in the spread in the full width half maximum (FWHM) and peak asymmetry read-outs of the LSPR assay. For the mannose-functionalized GNRs of the present invention it has been observed that for good results in the MBL detection assay, the mannose-functionalized GNRs should have FWHM between 95-120 nm and peak asymmetry between 0.95 and 1.25 and preferably a peak area between 100 and 125 nm$^2$ as demonstrated by the experimental results herein after. Hence in a particular embodiment according to the present invention the mannose functionalized GNRs are characterized in having an FWHM between 95-120 nm, peak asymmetry between 0.95 and 1.25, and a peak area between 100 and 125 nm$^2$.

In an even further embodiment the GNRs used are monodisperse long GNRs with an aspect ratio of about and between 3.0 to 4.5; more in particular of about and between 3.4 to 4.0; even more in particular of about and between 3.6 to 3.8. A perfectly monodispersed distribution means that all the nanoparticles have the very same geometry, this is the ideal condition, as a collection of identical particles is expected to be absolutely homogeneous in terms of typically size- and shape-dependent physico-chemical properties used in the localized surface plasmon resonance (LSPR) MBL binding assay of the invention. The polydispersity index (Pdl) is typically used to express the average uniformity of a (nano)particle solution. The Pdl is defined as the square of the standard deviation of the nanoparticle diameter distribution divided by the mean nanoparticle diameter. Larger Pdl values correspond to a larger size distribution in the nanoparticle sample. A sample is considered monodisperse when the Pdl value is less than 0.1. Alternatively, the polydispersity can also be assessed based on FWHM and peak asymmetry read-outs of the LSPR assay. Also, for FWHM and peak asymmetry (from UV-Vis peak analysis) a sample is considered monodisperse when their indices (the square of the standard deviation divided by the mean) are below 0.1.

When considering the application of the mannose-functionalized GNRs in an MBL detection assay, one further factor influencing the stability of the GNRs in the bodily fluid samples, is the surface coverage of the nanorods with the thiol-PEG-based (glyco)polymer, i.e. with the amount of mannose functionalized thiol-polyethylene glycol (PEG) based polymer linkers on the surface of the nanorods, hereinafter also referred to as mannose-thiol-polyethylene glycol (PEG) based polymer. A coverage of at least 3 molecules per nm$^2$ being required. In particular, the mannose-functionalized GNRs have a surface coverage, hereinafter also referred to as grafting density from 3 to about 5 of mannose-functionalized thiol-polyethylene glycol (PEG) based polymer linkers on the surface of the nanorods, more in particular a grafting density of about 4, as obtained by Anthrone-sulfuric acid and High-performance anion-exchange chromatography-pulsed amperometric detection (HPAEC-PAD).

In a second aspect the present invention provides a colloidal monodisperse suspension, in particular in water, of the mannose-functionalized GNRs as herein described. It has been found that the mannose-functionalized GNRs as herein described result in a stable suspension with a long shelf life (6 months to 1 year).

In a third aspect the present invention provides the use of the mannose-functionalized GNRs according to the invention and as herein provided, in a Mannose-binding lectin (MBL) binding assay, more in particular in a single-step MBL binding assay. In one embodiment, the present invention provides the use of the mannose-functionalized GNRs according to the invention and as herein provided, in a localized surface plasmon resonance (LSPR) MBL binding assay.

In a fourth aspect the present invention provides a localized surface plasmon resonance (LSPR) MBL binding assay comprising the use of the mannose-functionalized GNRs as herein provided. As mentioned herein before, it has been an object of the present invention to provide a simple assay to enable direct MBL measurement in a test sample, in particular a bodily fluid sample. As will become apparent from the examples hereinafter, the present inventors have been successful in realizing a single-step LSPR MBL binding assay that can be performed, even in undiluted serum samples.

Thus, in a particular embodiment the LSPR MBL binding assay according to the invention comprises the step of contacting the mannose-functionalized GNRs according to any one of the embodiments herein provided with a test sample; in particular an undiluted or diluted serum sample. In contacting the mannose-functionalized GNRs with the test sample, the former can simply be added directly to the sample or as a colloidal suspension, preferably the mannose-functionalized GNRs are added as a colloidal suspension in water, more preferably as a colloidal suspension in water at a final concentration of the mannose-functionalized GNRs of about and between 5 to 50 $\mu$g.mL$^{-1}$; in particular of about and between 7 to 30 $\mu$g.mL$^{-1}$; more in particular of about and between 10 to 20 $\mu$g.mL$^{-1}$; even more in particular at a final concentration of the mannose-functionalized GNRs of about 15 $\mu$g.mL$^{-1}$;

In an embodiment the LSPR MBL binding assay comprises recording the UV-VIS absorption spectra of mannose-functionalized GNRs; in particular between $\lambda$ = 400-1000 nm. It is common general knowledge that in an LSPR assay the peak maxima of the absorption spectra are being used to determine peak shifts resulting from binding of materials to the GNRs. In the instant case, the peak shifts result from MBL binding to the mannose-functionalized GNRs. Peak maxima can be determined from the zero crossings of the derivative of the absorption spectra, wherein the latter can eventually undergo a smoothing treatment, such as for example using a Savitzky-Golay filter or fitting of the observed spectrum to a mathematical function, for example a Gaussian or Voigt profile

Hence, in an embodiment the LSPR MBL binding assay comprises determining the LSPR peak shift over contact time with the test sample. Contact times as short as a couple of minutes are sufficient to observe a peak shift in the absorption spectra of the mannose-functionalized GNRs according to the invention. In an embodiment the contact time of the test sample with the mannose-functionalized GNRs is at least 2, 3, 4, 5, 6, 7, 8, 9, 10 minutes, in particular up to 20, 30, 40, 50, 60 minutes; more in particular the contact time of the test sample with the mannose-functionalized GNRs is about 15 minutes.

In the LSPR MBL binding assay according to the invention the change (shift) in LSPR peak position over time, is preferably expressed relative to the peak position at the start of the assay.

BRIEF DESCRIPTION OF THE DRAWINGS

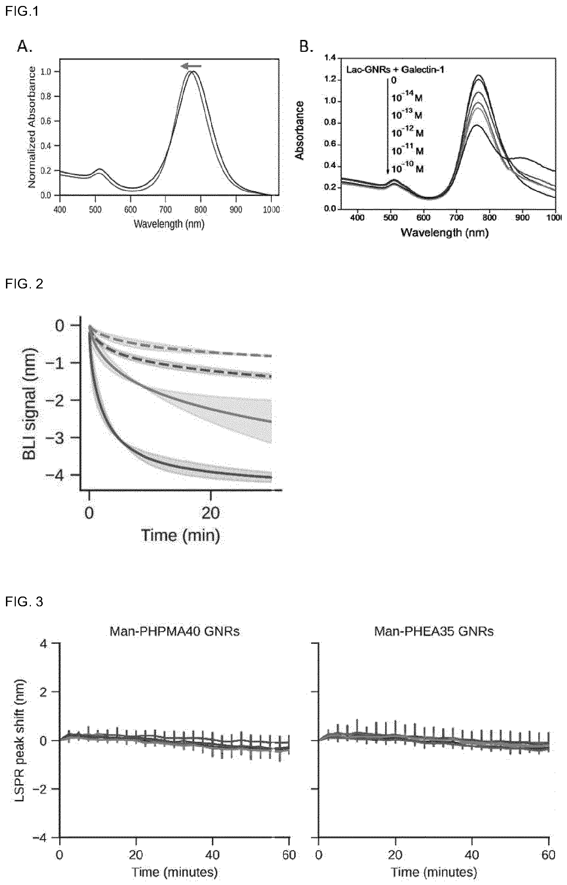

Figure 1. A) Representative example of LSPR peak wavelength shift for the mannose-functionalized GNRs used in the assay of the invention. B) Decrease in absorption spectra of Lac-GNRs after addition of galectin-1 with concentrations from 0 to 10$^{-10}$ M taken from Figure 2 (B) in Zhao et al.

Figure 2. Biolayer Interferometry (BLI) interaction assay of two concentrations (60 $\mu$g.mL$^{-1}$ upper line and 100 $\mu$g.mL$^{-1}$ lower line) of Man-PHEA35 (solid line) and Man-PHPMA40 (dashed line) on biotinylated-rhMBL immobilized on the surface of SAX2 biosensor tips in DPBS. The measurements were performed in duplicate. No shift in the BLI signal was observed for the controls using another biotinylated lectin or polymers without the sugar end group (e.g. PFP-PHPMA40 and PFP-PHEA35).

Figure 3. Response of Man-PHPMA40 GNRs (left) and Man-PHEA35 GNRs (right) to nMBL in human serum. A) The lines indicate the LSPR peak shift of the two glycopolymer-coated GNRs in serum as a function of nMBL concentration over time (N=3, mean +/- SD).

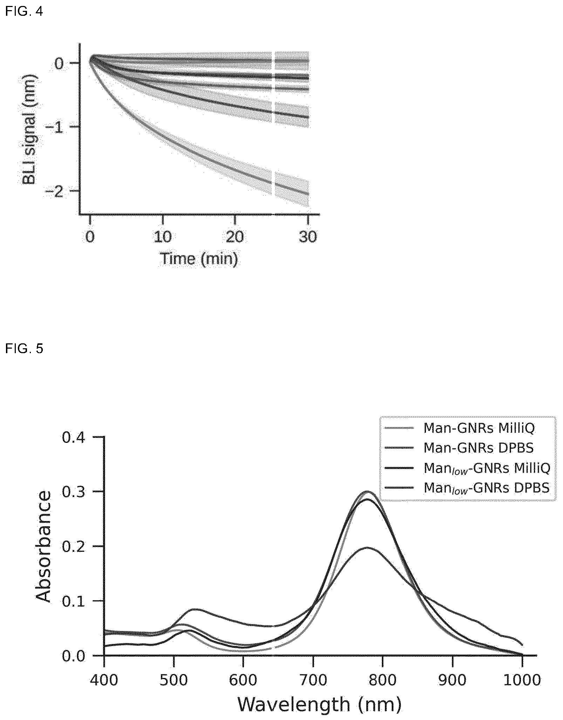

Figure 4: BLI interaction assay of several Man:COOH-coated GNRs ratios on biotinylated-rhMBL immobilized on the surface of SAX2 biosensor tips in DPBS. The measurements were performed in duplicate. No BLI signal has been observed for the controls. From top to bottom increasing concentrations of mannose (0%, 10%, 30%, 50%, 70%, 90%, 100%).

Figure 5. UV-Vis absorption spectra and pictures of Man-GNRs and Man$_{low}$-GNRs. A) Representative example out of three replicate measurements of UV-Vis absorption spectra of Man-GNRs and Man$_{low}$-GNRs in MilliQ and DPBS.

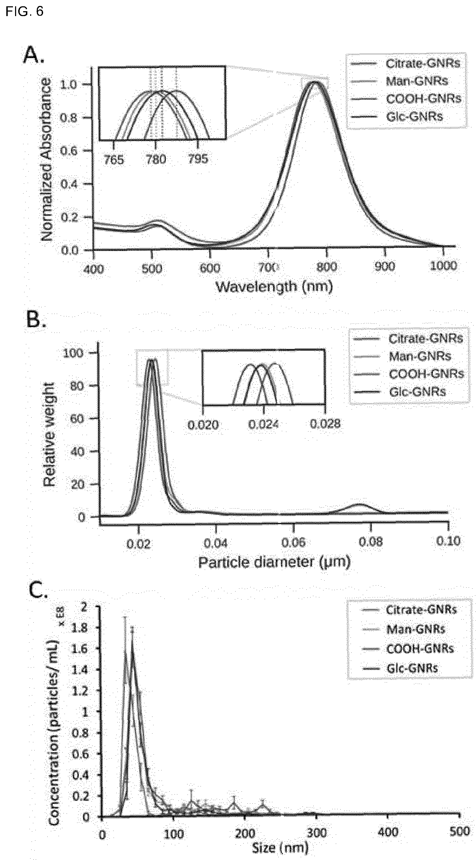

Figure 6. Glyconanoparticle characterisation. Representative example out of three replicate measurements of: (A) UV-Vis absorption spectra (Inset: zoomed view on the LSPR peak bands); (B) DCS (Inset: zoomed view of the peaks of the size distribution); (C) NTA of citrate-GNRs, Man-GNRs, Glc-GNRs and COOH-GNRs;

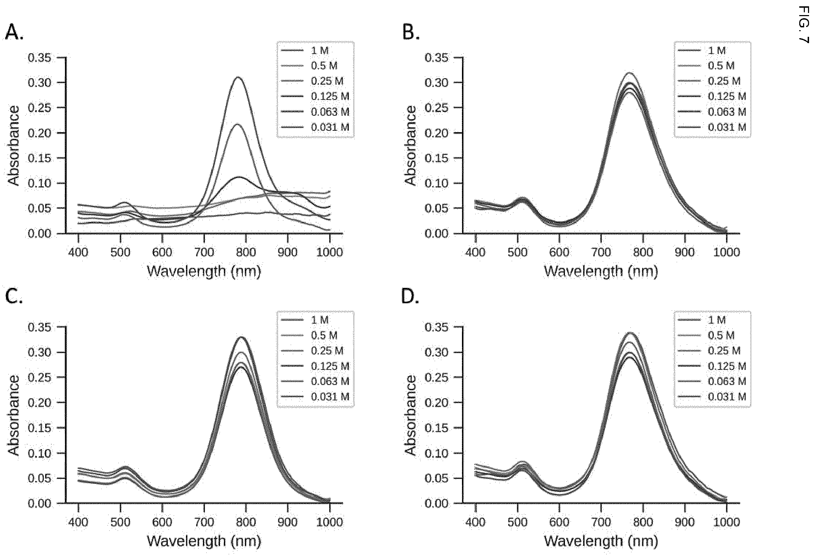

Figure 7. Salt titration assay. UV-Vis spectra of: (A) citrate-GNRs, (B) Man-GNRs, (C) COOH-GNRs, (D) Glc-GNRs upon incubation with different concentrations of NaCl (1, 0.5, 0.25, 0.125, 0.063, 0.031 M). A representative example out of three replicate measurements is shown.

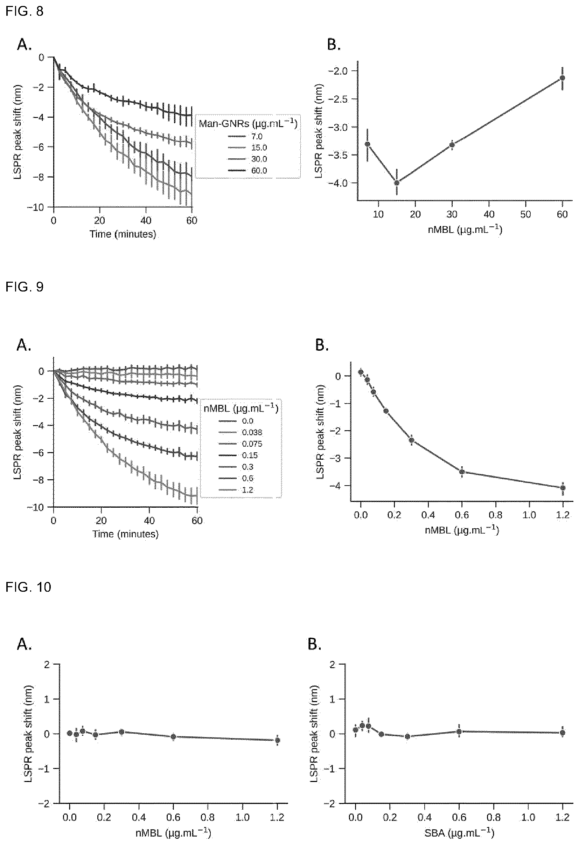

**Figure 8.** Response of different concentrations of Man-GNRs to nMBL (1.2 $\mu$g.mL$^{-1}$) in human serum. (A) The lines indicate the LSPR peak shift of Man-GNRs in serum as a function of Man-GNRs concentration over time. B) LSPR wavelength peak shift after 15 minutes is expressed relative to the peak position at the start of the injection (N=4, mean +/- SD).

**Figure 9.** Response of Man-GNRs to nMBL in human serum. A) The lines indicate the LSPR peak shift of Man-GNRs in serum as a function of nMBL concentration over time. B) LSPR wavelength peak shift after 15 minutes, relative to the peak position at the start of the injection (N=4, mean +/- SD).

**Figure 10.** Control conditions of COOH-GNRs and Man-GNRs in human serum. (A) LSPR peak shift of COOH-GNRs as a function of nMBL concentration in serum, determined by UV-Visible spectroscopy. (B) The same analysis was performed for Man-GNRs in MBL-depleted serum spiked with different concentrations of soybean agglutinin (SBA). All samples were analysed in triplicate every 2.5 minutes for 2 hours (mean +/- SD). Here we show the results after 15 minutes.

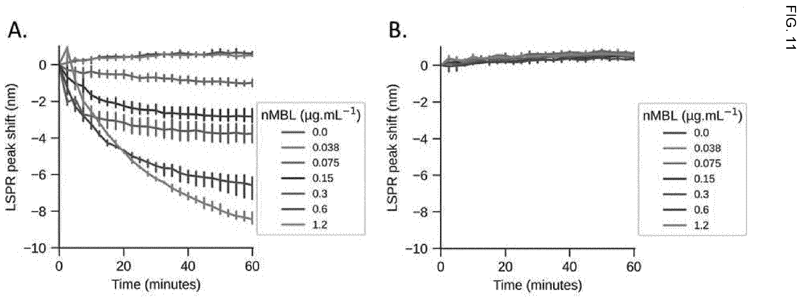

**Figure 11.** (A) Response of Man-GNRs to nMBL in human serum using a 96-well plate with a total volume of 200 $\mu$l. The lines indicate the LSPR peak shift of Man-GNRs in serum as a function of nMBL concentration over time. The samples were analysed in triplicate (mean +/-SD). (B) Control conditions of COOH-GNRs as a function of nMBL concentration, determined by UV-Visible spectroscopy in serum. The samples were analysed at least in duplicate (mean +/- SD).

## DETAILED DESCRIPTION OF THE INVENTION

In an earlier study lactose-functionalized gold nanorods were shown to be efficient biosensors to detect cancerous conditions based on the unique surface plasmon resonance properties of GNRs and high specificity of lactose to the galectin-1 cancer biomarker. A trace concentration of galectin-1 can be detected and the good specificity of Lac-GNRs to galectin-1 either in aqueous solutions or in the complex and heterogeneous serum specimens is showed (Zhao Y, Tong L, Li Y, et al. Lactose-Functionalized Gold Nanorods for Sensitive and Rapid Serological Diagnosis of Cancer. ACS Appl Mater Interfaces. 2016;8(9):5813-5820. doi:10.1021/acsami.5b11192).

As will be further detailed herein after, in the assay of the invention a thiol-PEG based polymer is also being used, in the assay of the invention, the mannose-functionalized GNRs differ in comprising an alkane chain between PEG and the thiol group. It is generally accepted that the inclusion of such an alkane chain moiety contributes to tighter packing and improved ordering of the polymer chains on gold surfaces. Surprisingly, the presence of the alkane linker provided stability of the nanoparticles in the presence of the biomarker MBL, which is a key challenge in the design of an LSPR binding assay. Where a stable suspension of mannose functionalized thiol-PEG based nanorods could be obtained, independent of the presence of the alkane linker, those comprising such linker were found to be protected from a ligand-induced aggregation.

In Zhao et al., the sensing principle relies on the aggregation of rods, which leads to the decrease of the LSPR peak intensity (at 770 nm) of nanorods after addition of the biomarker (Figure 1B). For this aggregation-based assay, the stability of the nanorods is less of an issue. Even though an aggregation due to instability of the rods is indistinguishable from the sensor response, it does not preclude the detection of the latter, this in contrast to the present assay that relies on the change in local refractive index of the medium caused by the binding of biomolecules to individual rods in suspension, which affects the localised surface plasmon resonance, and can be measured by a shift in the rods' optical spectrum (Figure 1A).

This difference is significant because:

- Stability of nanoparticles in suspension is accordingly a key challenge in the design of LSPR binding assays. In the case of aggregation-based assays, non-specific rod aggregation caused by interfering substances or assay kit degradation is indistinguishable from the sensor response. In contrast, in the case of our assay, the onset of aggregation can be assessed separately from the sensor signal and used as a quality control.
- The LSPR-shift-based assay of the present invention shows good linearity with the concentration of analyte, enabling a quantitative assay. Aggregation-based assays tend to have a non-linear dose-response behaviour. The study by Zhao et al. does not provide a calibration curve and is used for a binary determination of the biomarker level. In contrast, our LSPR-shift assay allows the quantitative determination of changes of biomarker levels within the physiological and pathological range. This limitation renders the aggregation-based readout unsuitable for the present application.

In view of the foregoing and with the objective of developing an LSPR binding assay, the earlier paper of Zhao et al., did not constitute a proper starting point, and the materials of the previously reported LSPR assay (Pancaro A, Szymonik M, Georgiou PG, et al. The polymeric glyco-linker controls the signal outputs for plasmonic gold nanorod biosensors due to biocorona formation. Nanoscale. 2021;13:10837-10848. doi:1 0.1039/d1nr01548f) were used instead. In this study the sensing performance of GNRs coated with galactosamine-modified polymeric linkers for the detection of the plant lectin soybean agglutinin were described. The polymers used in said paper (PHPMA40 and PHEA35) were modified with mannose for the detection of nMBL in human serum. Notwithstanding binding of the lectin with the mannose modified polymers as demonstrated by means of bio-layer interferometry (BLI) (Figure 2), the UV-Visible analysis (UV-Vis) did not show interaction with any concentration of nMBL (Figure 3).

These results demonstrate that the binding polymers have a significant impact, not only on the stability of the nanorods in suspension, but also on the LSPR signal. A further factor found to affect the LSPR binding assay performance appeared to be the density of the binding sites (grafting density). To study the impact of the grafting density in relation to nMBL binding, citrate-GNRs were functionalized with Man-EG$_6$C$_{11}$SH and COOH-OCH$_2$-EG$_6$C$_{11}$SH in several molar mass ratios (termed Man:COOH) to make a library of Man:COOH-coated GNRs (percentage of Man:COOH ratios: 90:10; 70:30; 50:50; 30:70; 10:90). In this case, the stability of the nanorods is achieved by all functionalized rods and confirmed by UV-Vis even in buffered saline solutions. Using the UV-Vis spectroscopy analysis to monitor the LSPR peak shift of Man:COOH-coated GNRs in human serum, no affinity for nMBL concentrations was observed for any of the aforementioned ratios. To check if MBL binding was retained, the same Man:COOH-coated GNRs samples were analysed using BLI (Figure 4). From about 15% mannose onwards the presence of said Man-GNRs led to ratios dependent BLI mannose binding signals which is attributed to the different grafting densities of mannose at the surface of gold nanorods. This further proves that even if there is interaction between mannose-coated GNRs and MBL, this does not automatically imply the ability to read out the signal via spectrophotometric detection. Finetuning of the nanoparticle functionalization, to ensure a high density of binding sites is critical to the performance of the assay. Only the citrate-GNRs that were fully functionalized with Man-EG$_6$C$_{11}$SH provided a detectable shift in the LSPR peak.

In the functionalization of the citrate-GNRs, the best results were obtained using an excess of the mannose comprising thiol-(glyco)polymers yielding a surface density of at least 3 molecules of the mannose comprising thiol-(glyco) polymers per nm$^2$. Lower amounts were found to result in a visible colorimetric shift in GNR solutions from red to purple indicating nanoparticle aggregation. This instability is worsened in buffers of higher ionic strength, making these formulations unsuitable for direct biosensing studies in bodily fluids such as serum.

As used herein, the mannose as part of the functionalized GNRs is meant to include the two different-sized rings, the pyranose (six-membered) form and the furanose (five-membered) form, both in $\alpha$ and $\beta$-configuration, and not restricted to monosaccharides but also includes oligosaccharides such as the disaccharides Man$p\,\beta$ 1-4 Man$p$, Man$p\,\beta$ 1-2 Man$p$, or Man$p\,\alpha$ 1-6 Man$p$.

Using the Man-GNRs nanoplasmonic sensors according to the invention in bodily fluids, the presence of bio(macro)molecules in such samples would inevitably lead to an interaction of said molecules with the nanoparticles, influencing the binding properties of the latter for lectin binding and corresponding LSPR detection. Still, and as demonstrated in the examples hereinafter, a dose-dependent signal could be obtained when contacting the Man-GNRs nanoplasmonic sensors according to the invention with human serum samples. Consequently, and as already mentioned herein before, the Man-GNRs nanoplasmonic sensors according to the invention can be used in biological fluids directly, including bodily fluids. Without being bound by theory, this could be due to the fact that the optical absorption maximum of the LSPR band lies within the near infrared region, where interfering absorption of bio-fluids is minimized.

The present invention accordingly provides a new nanoplasmonics-based biosensing platform for MBL detection in human serum. Man-GNRs biosensors were designed and fabricated for the detection of MBL based on the high affinity for mannose and unique LSPR properties of GNRs. The Man-GNRs biosensors showed to be specific for MBL detection even in a complex serum environment, and are able to detect concentrations as low as 160 ng.mL$^{-1}$ in just 15 minutes by using a microplate reader. This assay is easy to use and may have great potential in the further development of a point-of-care assay for MBL detection.

## EXAMPLES

### Materials

Citrate-stabilised gold nanorods (GNRs) of 10 nm width and 38 nm length ($\lambda$max = 780 nm) were purchased from Nanopartz (Loveland, USA/Canada) (3.8 mg.mL$^{-1}$). Pooled human serum (off-the-clot, type AB, male, sterile filtered) was purchased from Pan-biotech (Aidenbach, Germany); mannan-agarose beads from Sigma; COOH-OCH$_2$-(O-CH$_2$-CH$_2$)$_6$ (CH$_2$)$_{11}$SH (termed COOH-EG$_6$C$_{11}$SH where OCH$_2$-(O-CH$_2$-CH$_2$)$_6$ = EG$_6$ and (CH$_2$)$_{11}$ = C$_{11}$) from Prochimia (Gdynia,Poland); Gibco™ Dulbecco's phosphate buffered saline (DPBS) with calcium and magnesium, termed DPBS, from fisher scientific (Massachusetts, USA).

Synthetic mannose and glucose derivatives, termed Man-EG$_6$C$_{11}$SH and Glc-EG$_6$C$_{11}$SH respectively, were prepared as described previously by Midatech Pharma (Bilbao, Spain) (Barrientos AG, De la Fuente JM, Rojas TC, Fernández A, Penadés S. Gold glyconanoparticles: Synthetic polyvalent ligands mimicking glycocalyx-like surfaces as tools for glycobiological studies. Chem - A Eur J. 2003;9(9):1909-1921. doi:10.1002/chem.200204544 and dissolved in water).

All materials used for SDS-PAGE and western blot were obtained from Bio-Rad (California, USA) unless otherwise noted.

The preparation of the GNRs was conducted using Milli-Q grade water (resistivity of 18.2 m$\Omega$.cm at 25 °C, 4 ppb total organic carbon). Eppendorf Protein LoBind tubes were used for all experiments carried out in human serum.

## Methods

**(Glyco)polymer conjugation onto gold nanorods surface.** Citrate-stabilised gold nanorods were functionalized with thiol-(glyco)polymers (i.e, Man-EG$_6$C$_{11}$SH, Glc-EG$_6$C$_{11}$SH and COOH-OCH$_2$-EG$_6$C$_{11}$SH) dissolved in water. Briefly, 700 $\mu$L of GNR suspension in water (2 mg.mL$^{-1}$) was added dropwise to a solution of 15 $\mu$L of thiol-(glyco)polymers (100 mg.mL$^{-1}$), and incubated for 1 hour at room temperature on a shaker (30 rpm) in the dark. The conjugates (termed Man-GNRs, Glc-GNRs and COOH-GNRs, respectively) were sonicated for 1 min using a 40 KHz ultrasonic bath (Branson CPX1800H), centrifuged at 12000 g and 20 °C for 10 min, and the supernatant removed. This was followed by 3 cycles of washing by re-suspension in 1 mL water, centrifugation and the supernatant removed by pipetting. A Sigma 3-30KS centrifuge was used with 1.5 mL volume tubes for all preparative centrifugation. The particles were finally resuspended in 1 mL water and stored in polypropylene tubes at 4 °C until use.

**MBL-depleted human serum.** MBL-depleted human serum was prepared according to a method described previously (Rajagopalan R, Salvi VP, Jensenius JC, Rawal N. New insights on the structural/functional properties of recombinant human mannan-binding lectin and its variants. Immunol Lett. 2009;123(2):114-124. doi: 10.1016/j.imlet.2009.02.013). Briefly, a 5 mL column containing 2 mL of mannan-agarose as depleting beads was equilibrated with veronal buffer containing calcium chloride (3 mM) and magnesium chloride (10 mM). Human serum was passed through the column, and the flow-through was collected. The depletion of MBL was confirmed by Human MBL Quantikine ELISA (R&D Systems) showing $\geq$ 95% depletion efficiency.

**UV-Vis spectroscopy.** UV-Vis absorption spectra were acquired at 25 °C using a CLARIOstar Plus spectrophotometer (BMG Labtech, Ortenberg, Germany). All absorbance spectra were recorded between $\lambda = 400$-1000 nm with 1 nm resolution in clear, flat-bottomed 384-well plates (NUNC). The plates were shaken for 30 seconds at 100 RPM before each measurement. Results were smoothed using a Savitzky-Golay filter (order 4, window width 31) and full width half maximum (FWHM) is estimated. Peak maxima were determined from the zero crossings of the derivative of the smoothed data. Some results were normalized to better visualize the LSPR shift. All measurements were performed with at least three replicates (N $\geq$ 3).

Functionalised GNRs (5 $\mu$L of a 240 $\mu$g.mL$^{-1}$ suspension in water to reach a final concentration of 15 $\mu$g.mL$^{-1}$) were added to a series of different concentrations of rhMBL (0-2 $\mu$g.mL$^{-1}$) in 75 $\mu$L DPBS. For the assay carried out in human serum, 5 $\mu$L of different concentrations of Man-GNRs were added to 75 $\mu$L of serum containing 1.2 or 0.6 or 0.3 $\mu$g.mL$^{-1}$ native MBL (nMBL) to reach a final concentration of: 3.5 $\mu$g.mL$^{-1}$; 7 $\mu$g.mL$^{-1}$; 15 $\mu$g.mL$^{-1}$; 30 $\mu$g.mL$^{-1}$; 60 $\mu$g.mL$^{-1}$. The concentration of nMBL was confirmed using MBL Quantikine ELISA. Human serum containing 1.2 $\mu$g.mL$^{-1}$ nMBL was two-fold serially diluted in MBL-depleted human serum prepared as described above down to 0.038 $\mu$g.mL$^{-1}$. Absorbance spectra were recorded every 2.5 minutes after addition of 15 $\mu$g.mL$^{-1}$ (final concentration) of (glyco)polymer-coated GNRs.

**ELISA.** The 4.5 hour solid phase assay (Human MBL Quantikine ELISA Kit (DMBL00) from R&D Systems) was performed according to the instructions provided by the manufacturer. A buffered protein solution with preservatives (proprietary composition) is added to polystyrene microplate wells coated with a monoclonal antibody specific for human MBL. Serum samples diluted around 400 fold in calibrator diluent (buffered protein solution with preservatives) were added in duplicates according the manufacturer's guidelines. Following incubation for 2 h at room temperature on a horizontal orbital microplate shaker, the wells were washed three times with a wash solution (proprietary composition). A monoclonal antibody specific for human MBL conjugated to horseradish peroxidase with preservatives was added to each well followed by another incubation for 2 hours at room temperature on the shaker. After washing, the substrate solution (stabilized hydrogen peroxide and stabilized chromogen tetramethylbenzidine in equal volume) was added to each well and, following incubation for 30 minutes at room temperature on the benchtop in the dark, the reaction was stopped by adding 100 $\mu$L of stop solution (diluted hydrochloric acid solution). The optical density was read at 450 nm. Eight human MBL standards (from 10 to 0.156 ng.mL$^{-1}$ with two-fold serial dilution and 0 ng.mL$^{-1}$) provided with the kit and reconstituted with calibrator diluent, was assayed simultaneously.

**Ionic strength stability studies**. A solution of 1 M NaCl was two-fold serially diluted down to 0.031 M in a 384-well plate. Citrate-GNRs and conjugated GNRs were added to each well to a final concentration of 15 $\mu$g.mL$^{-1}$, mixed and

incubated at room temperature for 30 min, and then an absorbance spectrum was recorded.

**Nanoparticle tracking analysis** (NTA) was performed to measure GNRs' size before and after functionalisation using a NanoSight NS500 instrument (NanoSight, Wiltshire, UK) in scatter mode with a laser output of 75 mW at 532 nm (green) and sCMOS camera (camera level set at 15). All samples were analysed in water in duplicate at 25 °C and 3 videos of 60 seconds were recorded (1499 frames with 25 frames/second) for each sample. The number of particles/frame ranged from 40 to 80 for the GNR samples, and none were detected in the buffer control. The samples were diluted to $10^8$-$10^9$ particles.mL$^{-1}$ in MilliQ water prior to loading in the measurement chamber. For calibration, 100 nm polystyrene (PS) spheres were used. The mode was derived from a particle number concentration-based size distribution using the NTA software version 3.0. For the analysis, a detection threshold of 4 was used.

$\zeta$**-potential** was measured on a ZetaView PMX 120 instrument (Particle Metrix, Inning am Ammersee, Germany). Zeta potential standard ($Al_2O_3$) was used for verification of zeta potential. All samples were measured in 1 mM NaCl in 11 positions at 22 °C in triplicate. Zeta potential was automatically calculated from the corresponding electrophoretic mobilities ($\mu_E$) by using Henry's correction of the Smoluchowski equation. Software version 8.05.11 SP2 was used.

**Differential centrifugal sedimentation** (DCS) was performed to assess GNRs functionalization by measuring the particles' size distribution. For this a CPS DC24000 UHR disc centrifuge (Cambridge, UK) was used with a 8-24% (w/w) sucrose gradient and a rotation speed of 24000 RPM. Before each sample run, a size calibration step was performed by injecting polyvinyl chloride latex beads (239 nm) with particle density of 1.385 g.mL$^{-1}$. All measurements were performed with at least three replicates (N $\geq$ 3). As the settling of particles is shape-dependent, for GNRs a 'non-sphericity factor' (NSF) of 2.85 was applied in the CPS software. The disc centrifuge control system software automatically determines the particle diameter based on the particle detection time after injection.

**Anthrone-sulfuric acid** microplate assay was used as previously described (Leyva A, Quintana A, Sánchez M, Rodriguez EN, Cremata J, Sánchez JC. Rapid and sensitive anthrone e sulfuric acid assay in microplate format to quantify carbohydrate in biopharmaceutical products : Method development and validation. Biologicals. 2008; 36:134-141. doi: 10.1016/j.biologicals.2007.09.001). Briefly, the anthrone reagent was prepared right before analysis by dissolving 0.1 g of anthrone (0.1%) in 100 mL of concentrated sulfuric acid (98%), protected from light. The anthrone solution was added slowly to the microplate containing various concentrations of D-mannose in water (4-500 $\mu$g.mL$^{-1}$) to obtain a calibration curve; blank; Man-GNRs and COOH-GNRs in different concentrations. The microplate was incubated for 10 min at 4 °C and then 20 min at 100 °C. After cooling to room temperature, the absorbance at 620 nm was measured and the data were plotted against the concentration of D-mannose. All measurements were taken in duplicate.

**High-performance anion-exchange chromatography-pulsed amperometric detection (HPAEC-PAD)** was also used to analyse total saccharide concentration after acid hydrolysis to release mannose monosaccharides. Two concentrations of Man-GNRs and COOH-GNRs in water (0.5 and 2.25 mg.mL$^{-1}$) and monovalent mannose sugar (160 $\mu$g.mL$^{-1}$) as reference were hydrolysed in 4 M trifluoroacetic acid for 2 h and 30 min at 100 °C. Man-GNRs and COOH-GNRs were washed twice in 200 $\mu$L MilliQ via centrifugation (2000 g) to remove nanoparticles. The supernatant was collected and dried under nitrogen overnight.

The samples were redispersed in 400 $\mu$L milliQ and analysed using Dionex ICS-5000 (Thermo Scientific, Inc., Waltham, USA).

**Formation of protein corona.** The same volume of Man-GNRs and COOH-GNRs prepared in water, as described above, was added to 1 mL of human serum to reach different final concentrations (15 $\mu$g.mL$^{-1}$; 30 $\mu$g.mL$^{-1}$; 60 $\mu$g.mL$^{-1}$; 80 $\mu$g.mL$^{-1}$). They were prepared in duplicate and incubated at room temperature for 1 hour on a shaker at 40 rpm to allow formation of a biomolecule corona on the particles' surface. Moreover, 80 $\mu$g.mL$^{-1}$ of Man-GNRs was also prepared in MBL-depleted serum. The GNRs suspensions were centrifuged at 12000 g for 10 min at 15 °C, the supernatant was collected and analysed using Human MBL Quantikine ELISA Kit, while the pellet was resuspended in 1 mL of DPBS. The samples were washed three times by pipetting in 1 mL of DPBS and redispersed in 35 $\mu$L of DPBS for SDS-PAGE and Western Blot densitometry analysis.

**SDS-PAGE and western blotting.** After collection of nanoparticles-corona complexes (see above), they were resuspended in 35 $\mu$L DPBS and analysed after every washing step to determine the presence of the corona proteins. Equal volume of samples were heated at 95 °C in Laemmli sample buffer containing a final concentration of 69.45 mM Tris-HCl pH 6.8, 11.1% (v/v) glycerol, 1.1% LDS, 0.005% bromophenol blue and 355 mM 2-mercaptoethanol. Precision Plus Protein™ Unstained Protein Standards and Precision Plus Protein™ WesternC™ Blotting Standards (Bio-Rad) were used as markers. Samples were subjected to gel electrophoresis on a 4-20% mini PROTEAN TGX at 120 V. Sodium dodecyl sulphate polyacrylamide gel electrophoresis (SDS-PAGE) gels were imaged by the Stain-Free technology™, using a ChemiDoc XRS+ System (Bio-Rad) and quantified with the Image Lab Software (Bio-Rad). SDS-PAGE gels were blotted on a nitrocellulose membrane using the Trans-Blot Turbo™ RTA Mini Nitrocellulose Transfer Kit and Blotting System (Bio-Rad). Thereafter, membranes were blocked using EveryBlot blocking buffer (Bio-Rad) for 5 minutes and monoclonal mouse anti-human MBL antibody diluted in full-strength blocking buffer at final concentration of 1 $\mu$g.mL$^{-1}$ was added overnight at 4 °C with agitation. After three washing steps of 5 minutes each with PBS-T, an HRP-conjugated donkey anti-mouse IgG (1/10000, Jackson Immuno Research) and Precision protein streptavidin-HRP for the ladder (1/20000 Bio-Rad) were added for 1 hour at room temperature in full-strength block. Thereafter, membranes were washed in PBS-T and developed using the Clarity Western ECL Substrate kit according the manufacturer's guidelines. Membranes were imaged using a ChemiDoc XRS + System (Bio-Rad).

**Calculation of D-mannose density on GNRs** The amount of mannose per $nm^2$ of gold nanorods was calculated using the HPAEC-PAD results. Considering the molecular weight and the Avogadro number, we calculated the number of molecules of sugar present in 1 mg.mL$^{-1}$ of functionalized GNRs.

The volume of each GNR is calculated using the following equation:

$$Volume = \left((length - diameter) * \pi r^2\right) + \left(\frac{4}{3}\right)\pi r^3$$ and estimated to be 2722.7 nm$^3$.

Assuming a density equal to that of gold (19.32 g.cm$^{-3}$), the weight of each GNR is calculated (*density * volume* = 5.3 * 10$^{-17}$g).

Knowing the number of molecules of mannose present in 1 mg.mL$^{-1}$ of functionalized GNRs, the weight of each GNR and their surface area $(((2\pi r) * (length - diameter)) + 4\pi r^2 = 1193.8\ nm^2)$ we estimated 4 molecules.nm$^{-2}$ on every nanoparticle.

## RESULTS AND DISCUSSION

### Gold nanorods functionalization, stability study and characterization.

The lability of the citrate ions adsorbed on the citrate-gold nanoparticles surface and the high binding affinity of thiols to gold lead to a high surface coverage of polymers providing an ideal system for the study of molecules interaction. The surface grafting density is a key parameter producing different LSPR binding response to lectin interaction, for example higher ligand density induced gold nanoparticles aggregation upon addition of specific lectin in buffer. To analyse the impact of two grafting densities on glyco-conjugated GNR stability, citrate-GNRs (700 μL, 2 mg.mL$^{-1}$) were functionalized with 15 μL of 2 mg.mL$^{-1}$ and 100 mg.mL$^{-1}$ of Man-EG$_6$C$_{11}$SH. Whereas 100 mg.mL$^{-1}$ Man-EG$_6$C$_{11}$SH resulted in equally stable suspensions in both water and DPBS, the low grafting concentration (termed Man$_{low}$-GNR) resulted in a visible colorimetric shift in GNR solutions from red to purple indicating nanoparticles' aggregation (Figure 5 and Table 1). This colour change has previously been reported for the development of nano-diagnostic colorimetric assays (Niazi A, Jorjani O-N, Nikbakht H, Gill P. A nanodiagnostic colorimetric assay for 18S rRNA of Leishmania pathogens using nucleic acid sequence-based amplification and gold nanorods. Mol Diagn Ther. 2013;17(6):363-370. doi:10.1007/s40291-013-0044-5; Saber R, Shakoori Z, Sarkar S, Tavoosidana G, Kharrazi S, Gill P. Spectroscopic and microscopic analyses of rod-shaped gold nanoparticles interacting with single-stranded DNA oligonucleotides. IET nanobiotechnology. 2013;7(2):42-49. doi:10.1049/iet-nbt.2012.0009). In our work, this instability was enhanced in DPBS compared to water as dispersant, and did not allow MBL binding studies. Hence, the GNR conjugation explained in materials and method section (100 mg.mL$^{-1}$ of Man-EG$_6$C$_{11}$SH ) was used for further assay development in this study.

Table 1. Peak maxima, full width half maximum (FWHM) peak asymmetry and area of Man-GNRs and Man$_{low}$-GNR in MilliQ and DPBS (N=3, mean +/- SD).

|  | peak maximum (nm) | FWHM (nm) | Peak asymmetry | Peak area |
|---|---|---|---|---|
| Man-GNRs MilliQ | 780 $\pm$ 1 | 105 $\pm$ 1 | 1.20 $\pm$ 0.01 | 118 $\pm$ 0.1 |
| Man-GNRs in DPBS | 778 $\pm$ 1 | 112 $\pm$ 1 | 1.06 $\pm$ 0.02 | 119 $\pm$ 0.1 |
| Man$_{low}$-GNR in MilliQ | 775 $\pm$ 3 | 121 $\pm$ 2 | 1.19 $\pm$ 0.16 | 137 $\pm$ 11 |
| Man$_{low}$-GNR in DPBS | 774 $\pm$ 5 | 120 $\pm$ 5 | 1.42 $\pm$ 0.06 | 129 $\pm$ 10 |

Citrate-stabilized GNRs were mixed with Man-EG$_6$C$_{11}$SH, Glc-EG$_6$C$_{11}$SH or COOH-OCH$_2$-EG$_6$C$_{11}$SH to produce (glyco)polymer-coated GNRs (termed Man-GNRs, Glc-GNRs and COOH-GNRs, respectively). Excess glycopolymer was removed by three centrifugation and resuspension cycles. The physico-chemical properties of the resulting nanoparticles were characterized in MilliQ water by UV-Vis spectroscopy, DCS, $\zeta$-potential measurement and NTA, (Figure 6 and Table 2). UV-Vis spectroscopy revealed a red shift of the longitudinal LSPR band for the glycopolymer-coated GNRs, $\zeta$-potential confirmed the successful attachment of the glycopolymers to the particle surface, while DCS and NTA analyses indicated an increase in particle size compared with the citrate-GNRs, as expected (see Table 2).

**Table 2**. Characterisation of glycopolymer-coated GNRs. UV-Vis LSPR peak (nm), $\zeta$-potential (mV), and peak diameter (nm) by DCS and mode (nm) by NTA of citrate-GNRs and (glyco)polymer-coated GNRs (N=3, mean +/- SD).

|  | UV-Vis LSPR peak (nm) | $\zeta$-Potential (mV) | DCS Peak diameter (nm) | NTA size Mode (nm) |
|---|---|---|---|---|
| **Citrate-GNRs** | 769 $\pm$ 1 | - 45.2 $\pm$ 0.5 | 24.9 $\pm$ 0.1 | 40.6 $\pm$ 0.5 |
| **Man-GNRs** | 772 $\pm$ 1 | - 37.8 $\pm$ 0.1 | 24.0 $\pm$ 0.1 | 46.0 $\pm$ 0.2 |
| **COOH-GNRs** | 778 $\pm$ 1 | -41 $\pm$ 1 | 23.3 $\pm$ 0.1 | 48.5 $\pm$ 0.6 |
| **Glc-GNRs** | 773 $\pm$ 1 | - 37.5 $\pm$ 0.4 | 23.9 $\pm$ 0.2 | 46.8 $\pm$ 0.2 |

To analyze the coating density of Man-GNRs, the amount of carbohydrate ligands coupled to gold nanorods was determined by a colorimetry method using anthrone-sulfuric acid. This is one of the most commonly used and efficient techniques for the quantitative analysis of carbohydrates, and has been adopted in glyconanoparticle analysis. The amount of mannose attached to GNRs was subsequently derived from the calibration curve and estimated to be 31 $\pm$ 5 $\mu$g.mL$^{-1}$ (175 $\pm$ 28 $\mu$M) for 1 mg.mL$^{-1}$ (0.032 $\mu$M) of Man-GNRs. The same method was employed in another study to measure lactose on Lac-GNRs finding a concentration of 110 $\mu$g.mL$^{-1}$ for 2 mg.mL$^{-1}$ of Lac-GNRs (Zhao Y, Tong L, Li Y, et al. Lactose-Functionalized Gold Nanorods for Sensitive and Rapid Serological Diagnosis of Cancer. ACS Appl Mater Interfaces. 2016;8(9):5813-5820. doi:10.1021/acsami.5b11192). COOH-GNRs was used as reference sample.

A non-colorimetric method was also applied to estimate the number of carbohydrate per nanoparticle that allows separation of gold nanorods before the quantification. HPAEC-PAD is a well-established selective technique with sensitive detection for carbohydrate analysis and was previously employed to quantify the effective amount of sugar linked to gold nanoparticles (Pitirollo O, Micoli F, Necchi F, et al. Gold nanoparticles morphology does not affect the multivalent presentation and antibody recognition of Group A Streptococcus synthetic oligorhamnans. Bioorg Chem. 2020;99(January):103815. doi:10.1016/j.bioorg.2020.103815). Man-GNRs and COOH-GNRs showed results close to the anthrone-sulfuric acid colorimetric assay: an average of 26.6 $\pm$ 0.4 $\mu$g.mL$^{-1}$ (148 $\pm$ 3 $\mu$M) of mannose for Man-GNRs and no sugar detected for the control. Moreover, monovalent mannose sugar (160 $\mu$g.mL$^{-1}$) treated and analysed in the same way was used as internal reference, leading to a concentration of 155 $\mu$g.mL$^{-1}$ proving the reliability of the procedure.

The mannose-PEG grafting density was calculated and estimated to be around 4 molecules per nm$^2$ in agreement with the values reported in literature in which PEG ligands and spherical gold nanoparticles reported a grafting density of 3.5 molecules per nm$^2$ obtained by quantitative proton nuclear magnetic resonance and thermogravimetric analysis, and a surface coverage from 4.3 to 6.3 molecules nm$^2$ by inductively coupled plasma mass spectrometry. (Hinterwirth H, Kappel S, Waitz T, Prohaska T, Lindner W, Lämmerhofer M. Quantifying thiol ligand density of self-assembled monolayers on gold nanoparticles by inductively coupled plasma-mass spectrometry. ACS Nano. 2013;7(2):1129-1136. doi:10.1021/nn306024a; Retout M, Brunetti E, Valkenier H, Bruylants G. Limits of thiol chemistry revealed by quantitative analysis of mixed layers of thiolated-PEG ligands grafted onto gold nanoparticles. J Colloid Interface Sci. 2019;557:807-815. doi:10.1016/j.jcis.2019.09.047).

The stability of the Man-GNRs suspensions was studied using a sodium chloride titration assay. The nanoparticles' colloidal stability under physiological saline concentrations (~0.150 M NaCl) is a prerequisite for any biomedical applications and a further indication of a successful functionalization. UV-Vis spectroscopy analysis of the LSPR peak, which is highly sensitive to aggregation, showed aggregation of citrate-GNRs above 0.125 M NaCl and no changes of all the functionalized samples that remained as stable dispersions up to 1 M NaCl (Figure 7). This improvement in stability confirms the suitability of the functionalized rods for testing in physiological salt levels removing false positives/negatives due to unwanted aggregation.

**Recombinant human MBL detection in buffer**

MBL detection was first tested in DPBS using rhMBL. The particles (15 $\mu$g.mL$^{-1}$ ) were incubated with rhMBL concentrations between 0 and 2 $\mu$g.mL$^{-1}$ in DPBS and UV-Vis spectra recorded to observe changes in the LSPR peaks. The plant lectin SBA which has high affinity for N-acetylgalactosamine (GalNAc) but does not exhibit affinity towards mannose, as well as particles with non-glycosylated polymer (COOH-GNRs) or with glucose moiety (Glc-GNRs) were used as controls.

Notwithstanding the fact that the Man-GNRs were unstable and aggregated in less than 15 minutes upon addition of rhMBL concentration $\geq$ 0.5 $\mu$g.mL$^{-1}$, as seen by broadening of the LSPR peak, increase of the peak area and a decrease in the overall absorbance (data not shown), the samples still showed a binding response (red LSPR shift) without aggregation (i.e. no difference in full width half maximum and peak area) at rhMBL concentrations > 0.063 $\mu$g.mL$^{-1}$ and < 0.5 $\mu$g.mL$^{-1}$ (Table 3).

In further control experiments, no change in the spectrum was observed after injection of the control SBA, in the presence of glucose (Glc-GNRs) or with the carboxyl polymer (COOH-GNRs), indicating that the responses observed are due to specific carbohydrate-lectin interactions (data not shown).

**Table 3.** Peak maxima, full width half maximum (FWHM) peak asymmetry and area of Man-GNRs with different concentrations of rhMBL in DPBS after 15 minutes. All measurements were performed with at least three replicates (mean +/- SD).

| rhMBL ($\mu$g.mL$^{-1}$) | peak maxima (nm) | FWHM (nm) | Peak asymmetry | Peak area |
|---|---|---|---|---|
| 0 | 777 $\pm$ 0.2 | 112 $\pm$ 1 | 1.06 $\pm$ 0.01 | 118 $\pm$ 1 |
| 0.063 | 777 $\pm$ 0.2 | 111 $\pm$ 1 | 1.08 $\pm$ 0.01 | 118 $\pm$ 1 |
| 0.125 | 779 $\pm$ 0.1 | 111 $\pm$ 1 | 1.04 $\pm$ 0.02 | 117 $\pm$ 1 |
| 0.25 | 780 $\pm$ 0.3 | 111 $\pm$ 1 | 0.95 $\pm$ 0.04 | 117 $\pm$ 2 |
| 0.5 | 780 $\pm$ 0.7 | 137 $\pm$ 3 | 0.89 $\pm$ 0.02 | 143 $\pm$ 2 |
| 1 | 782 $\pm$ 0.6 | 161 $\pm$ 6 | 1.19 $\pm$ 0.06 | 160 $\pm$ 5 |
| 2 | 766 $\pm$ 6.7 | 178 $\pm$ 2 | 1.35 $\pm$ 0.21 | 174 $\pm$ 1 |

## MBL binding assay in human serum

To evaluate native MBL (nMBL) detection by binding onto the Man-GNRs nanoplasmonic sensors, human serum from healthy donors was used. In biofluids such as human serum, nanoparticles are exposed and interact with many bio(macro)molecules, leading to the formation of a "biomolecular corona", which may influence the physico-chemical properties and impact the binding properties of the nanoparticles.

The interaction between nMBL and GNRs is dependent on the concentration of both species. For this reason, before testing different nMBL concentrations in human serum, we evaluated the rods concentration to achieve the highest performance in terms of LSPR shift and reproducibility. Different Man-GNRs concentrations were employed in undiluted human serum, which contained a concentration of 1.2 $\mu$g.mL$^{-1}$ nMBL as determined by a commercially available Human MBL Quantikine ELISA Kit. The results showed that the highest performance was reached using a Man-GNRs concentration of 15 $\mu$g.mL$^{-1}$ (Figure 8, A and B). Lower rod concentrations (3.5 $\mu$g.mL$^{-1}$)showed high instability even in MBL-depleted serum (data not shown), probably due to physical aggregation of rods or due to low absorbance readings, while higher concentrations led to smaller LSPR shift due to the high amount of Man-rods in relation to nMBL. The optimal GNR concentration was also evaluated in human serum containing lower concentrations of nMBL (0.6 $\mu$g.mL$^{-1}$ and 0.3 $\mu$g.mL$^{-1}$). In conclusion, we showed that the concentration of rods strongly influences the biosensor performance. A constant rods concentration of 15 $\mu$g.mL$^{-1}$ was used further in this study.

Using the optimized Man-GNRs concentration, we next evaluated the limit of detection of nMBL binding in serum. For this, human serum containing 1.2 $\mu$g.mL$^{-1}$ of nMBL was two-fold serially diluted down to 0.038 $\mu$g.mL$^{-1}$ using MBL-depleted serum (Kawasaki T. Structure and biology of mannan-binding protein, MBP, an important component of innate immunity. Biochim Biophys Acta. 1999;1473(1):186-195. doi:10.1016/s0304-4165(99)00178-6; Kawasaki N, Kawasaki T, Yamashina I. Isolation and characterization of a mannan-binding protein from human serum. J Biochem. 1983;94(3):937-947. doi:10.1093/oxfordjournals.jbchem.a134437) to keep other serum constituents at physiological levels. Serum nMBL levels and the successful MBL-depletion ($\geq$ 95% depletion efficiency) were measured with ELISA.

We monitored the LSPR peak over time following the addition of Man-GNRs using a microplate reader. Man-GNRs showed dose-dependent LSPR blue shifts in serum (Figure 9, A). The LSPR shift can be observed after just 15 minutes (Figure 9, B). The limit of detection (LOD) for native MBL detection in human serum using glyco-coated GNRs is 0.1 $\mu$g.mL$^{-1}$. The response of Man-GNR to nMBL in serum was found to be reproducible across four different coupling reactions from 2 batches of GNRs (N=4) as showed in Figure 9. LSPR shift of 0.14 $\pm$ 0.28 at 15 minutes (i.e. no interaction) was observed in MBL-depleted serum (0.074 $\pm$ 0.004 $\mu$g.mL$^{-1}$ of nMBL) or using COOH-GNRs without glycan modification (Figure 10, A). In further control experiments, addition of different SBA concentration to MBL-depleted serum did not lead to any response, confirming the selective nMBL recognition in complex media (Figure 10, B).

Due to the presence of several lectins in serum with affinity for glucose, Glc-GNRs showed interaction in MBL-depleted serum, as well as serum containing different concentrations of nMBL, proving that the depletion strategy did not significantly affect the lectins without affinity for mannose. It was found that the binding of serum lectins to Glc-GNRs over time is not dependent of MBL dose (data not shown).

Since the 96-well format is compatible with various spectrophotometers and automated equipment, and it is most commonly used by laboratory technicians and researchers, we demonstrated the reliability of our assay by using a 96-well plate with a volume of 200 μL. The results are consistent with the same assay carried out in a 384-well plate (Figure 11, A and B).

**Quantification of MBL binding to mannose-conjugated gold nanorods**

Different methods were used to confirm the specificity of the interaction of Man-GNRs to nMBL in human serum and estimate the amount of lectin bound to every nanoparticle. Considering the surface area of the rod in relation to the area of the carbohydrate recognition domains of MBL (estimated via Pymol based on the protein structure PDB ID: 1HUP), the maximum amount of lectin molecules was estimated to be about 25, without considering the steric hindrance. The reliability of this value was experimentally tested using ELISA and western blot densitometry.

A method commonly used for determining the amount of conjugated protein on the surface of nanoparticles is to measure the protein remaining in the supernatant after bioconjugation. We used ELISA to determine the concentration of unbound nMBL in the collected supernatant after incubation of several Man-GNRs and COOH-GNRs concentrations (15, 30, 60, 80 μg.mL$^{-1}$) prepared in duplicate in human serum (containing 1.2 μg.mL$^{-1}$ nMBL), centrifugation and washing steps. The amount of nMBL bound to rods are shown in Table 4.

The concentration of bound nMBL was also estimated using western blot followed by densitometry analysis. Man-GNRs and COOH-GNRs (15; 30; 60; 80 μg.mL$^{-1}$) were incubated in human serum containing native MBL (1.2 μg.mL$^{-1}$), and the nanoparticle-corona complexes were isolated by a total of three centrifugation steps, resuspended in DPBS and the pellet after every centrifugation step was characterised by SDS-PAGE.

The presence of nMBL bound on the Man-GNR surface was identified by western blot (data not shown). The results showed the presence of nMBL around Man-GNRs after every washing step (as expected) and no traces of the lectin in the control COOH-GNRs. No nMBL was detected for Man-GNRs incubated in MBL-depleted serum, confirming that the depletion strategy was specific for mannose-binding lectins (data not shown). Moreover, the concentration of nMBL present in the hard corona was estimated in a second western blot loaded with different sample dilutions using a densitometry analysis against a known amount of rhMBL The concentration calculated showed in Table 4 are in good agreement with the ELISA results.

**Table 4.** Amount of nMBL bound to Man-GNRs in human serum, determined by ELISA and western blot (WB) densitometry analysis. Man-GNRs samples were prepared twice and a duplicate analysis was performed for each sample (mean +/- SD). MBL bound (%) according to ELISA and WB compared to the concentration of nMBL in human serum (1.2 μg.mL$^{-1}$).

| Man-GNRs (μg.mL$^{-1}$) | nMBL bound (ng.mL$^{-1}$ and %) by ELISA | nMBL bound (ng.mL$^{-1}$ and %) by WB |
|---|---|---|
| 15 | 210 $\pm$ 30 (18%) | 227 $\pm$ 6 (19%) |
| 30 | 430 $\pm$ 40 (36%) | 370 $\pm$ 20 (31%) |
| 60 | 810 $\pm$ 20 (68%) | 740 $\pm$ 40 (61%) |
| 80 | 955 $\pm$ 34 (80%) | 890 $\pm$ 20 (74%) |

The change in the LSPR signal is proportional to the amount of bound protein. Knowing the amount of nMBL bound (Table 4), the area of Man-GNRs nanoparticles used and the corresponding LSPR shift after one hour, we calculated the sensitivity of this biosensor. The result showed that approximately 14.5 ng of protein are bound per cm$^2$ rod area for one LSPR nm shift. Expressed differently, and taking into account that for all concentration of the control COOH-GNRs the level of bound MBL was below 60 ng.ml$^{-1}$ (data not shown) an average number of 11 to 13 nMBL monomers (formed by three identical polypeptides chains) binding onto each functionalized nanorod can be derived.

Starting from Man-GNRs conjugate suspensions with optimized stability, HPAEC-PAD and anthrone-sulfuric acid assay were employed to quantify the effective amount of sugar linked to GNRs. ELISA and western blot densitometry analysis were used to confirm the interaction of Man-GNRs to nMBL in human serum and estimate the amount of lectin bound to every nanoparticle. Man-GNRs showed to be specific for MBL detection even in a complex serum environment, and are able to detect concentrations as low as 100 ng.mL$^{-1}$ in just 15 minutes by using a microplate reader.

**Claims**

1.   Mannose-functionalized gold nanorods (GNRs) wherein the mannose is linked to the gold nanorods by a thiol-polyethylene glycol (PEG)-based polymer linker, **characterized in** comprising an alkane linker between the PEG and the thiol group, wherein the thiol is bound to the surface of the gold nanorods with a surface coverage of at least 3

molecules per nm$^2$.

2. The mannose-functionalized GNRs according to claim 1, wherein the thiol- PEG-based polymer linker is represented by the following formula (I) -S-(CH2)n-(OCH2CH2)m-O--S-alkyl-(OCH2CH2)m-O- (I) wherein n represents an integer from 2 to 20; and m represents an integer from 2 to 15.

3. The mannose-functionalized GNRs according to claim 2 wherein the GNRs have an aspect ratio of at least 2.

4. A colloidal monodisperse suspension of the mannose-functionalized GNRs according to any one of the preceding claims.

5. Use of the mannose-functionalized GNRs according to any one of the preceding claims in a Mannose-binding lectin (MBL) binding assay.

6. Use according to claim 5, wherein the MBL binding assay is a localized surface plasmon resonance (LSPR) MBL binding assay.

7. A localized surface plasmon resonance (LSPR) MBL binding assay comprising the use of the mannose-functionalized GNRs according to any one of claims 1 to 4.

8. The LSPR MBL binding assay according to claim 7, comprising the step of contacting the mannose-functionalized GNRs according to any one of claims 1 to 4 with a test sample; in particular a biological fluid sample, more in particular an undiluted or diluted serum sample.

9. The LSPR MBL binding assay according to claims 7 or 8, comprising recording the UV-Vis absorption spectra of mannose-functionalized GNRs; in particular recorded between $\lambda$ = 400-1000 nm, further in particular, where the sensing readout is based on the near infrared region between 700 and 900 nm.

10. The LSPR MBL binding assay according any one to claims 8 to 9, comprising determining a change in the LSPR peak position over contact time with the test sample.

11. The LSPR MBL binding assay according to claim 10, wherein the change in LSPR peak position is expressed relative to the peak position at the start of the assay.

**Patentansprüche**

1. Mannose-funktionalisierte Gold-Nanostäbchen (GNRs), wobei die Mannose mit den Gold-Nanostäbchen durch einen Thiol-Polyethylenglykol (PEG)-basierten Polymer-Linker verbunden ist, **dadurch gekennzeichnet, dass** er einen Alkan-Linker zwischen dem PEG und der Thiol-Gruppe umfasst, wobei das Thiol an die Oberfläche der Gold-Nanostäbchen mit einer Oberflächenabdeckung von mindestens 3 Molekülen pro nm$^2$ gebunden ist.

2. Mannose-funktionalisierte GNRs nach Anspruch 1, wobei der Thiol-PEGbasierte Polymer-Linker durch die folgende Formel (I) dargestellt wird -S-(CH2)n-(OCH2CH2)m-O- -S-alkyl-(OCH2CH2)m-O- (I) wobei n eine ganze Zahl von 2 bis 20 darstellt; und m eine ganze Zahl von 2 bis 15 darstellt.

3. Mannose-funktionalisierte GNRs nach Anspruch 2, wobei die GNRs ein Aspektverhältnis von mindestens 2 aufweisen.

4. Kolloidale monodisperse Suspension der Mannose-funktionalisierten GNRs nach einem der vorstehenden Ansprüche.

5. Verwendung der Mannose-funktionalisierten GNRs nach einem der vorstehenden Ansprüche in einem Bindungsassay eines Mannose-bindenden Lektins (MBL).

6. Verwendung nach Anspruch 5, wobei der MBL-Bindungsassay ein lokalisierter Oberflächenplasmonenresonanz-(LSPR)-MBL-Bindungsassay ist.

**7.** Lokalisierter Oberflächenplasmonenresonanz-(LSPR)-MBL-Bindungsassay, umfassend die Verwendung der Mannose-funktionalisierten GNRs nach einem der Ansprüche 1 bis 4.

**8.** LSPR-MBL-Bindungsassay nach Anspruch 7, umfassend den Schritt eines Kontaktierens der Mannose-funktionalisierten GNRs nach einem der Ansprüche 1 bis 4 mit einer Testprobe; insbesondere einer biologischen Flüssigkeitsprobe, weiter insbesondere einer unverdünnten oder verdünnten Serumprobe.

**9.** LSPR-MBL-Bindungsassay nach Anspruch 7 oder 8, umfassend ein Aufzeichnen der UV-Vis-Absorptionsspektren von Mannose-funktionalisierten GNRs; insbesondere aufgezeichnet zwischen $\lambda$ = 400-1000 nm, weiter insbesondere, wobei die Sensorauslesung auf dem Nahinfrarotbereich zwischen 700 und 900 nm basiert.

**10.** LSPR-MBL-Bindungsassay nach einem der Ansprüche 8 bis 9, umfassend das Bestimmen einer Änderung der LSPR-Peakposition im Laufe der Kontaktzeit mit der Testprobe.

**11.** LSPR-MBL-Bindungsassay nach Anspruch 10, wobei die Änderung der LSPR-Peakposition relativ zu der Peakposition zu Beginn des Assays ausgedrückt wird.

**Revendications**

**1.** Nanotiges d'or (GNR) fonctionnalisées par du mannose, dans lesquelles le mannose est lié aux nanotiges d'or par un lieur polymère à base de thiol-polyéthylène glycol (PEG), **caractérisée en ce qu'**elles comprennent un lieur alcane entre le PEG et le groupe thiol, dans lesquelles le thiol est lié à la surface des nanotiges d'or avec une couverture de surface d'au moins 3 molécules par nm$^2$.

**2.** GNR fonctionnalisées par du mannose selon la revendication 1, dans lesquels le lieur polymère à base de thiol-PEG est représenté par la formule (I) suivante -S-(CH2)n-(OCH2CH2)m-O- -S-alkyl-(OCH2CH2)m-O- (I)
dans lesquelles n représente un nombre entier allant de 2 à 20 ; et m représente un nombre entier allant de 2 à 15.

**3.** GNR fonctionnalisées par du mannose selon la revendication 2, dans lesquels les GNR ont un rapport de forme d'au moins 2.

**4.** Suspension monodispersée colloïdale des GNR fonctionnalisées par du mannose selon l'une quelconque des revendications précédentes.

**5.** Utilisation des GNR fonctionnalisées par du mannose selon l'une quelconque des revendications précédentes dans un essai de liaison de lectine liant le mannose (MBL).

**6.** Utilisation selon la revendication 5, dans laquelle l'essai de liaison de MBL est un essai de liaison de MBL par résonance plasmonique de surface localisée (LSPR).

**7.** Essai de liaison de MBL par résonance plasmonique de surface localisée (LSPR) comprenant l'utilisation des GNR fonctionnalisées par du mannose selon l'une quelconque des revendications 1 à 4.

**8.** Essai de liaison de MBL par LSPR selon la revendication 7, comprenant l'étape consistant à mettre en contact les GNR fonctionnalisées par du mannose selon l'une quelconque des revendications 1 à 4 avec un échantillon de test ; en particulier un échantillon de fluide biologique, plus particulièrement un échantillon de sérum non dilué ou dilué.

**9.** Essai de liaison de MBL par LSPR selon les revendications 7 ou 8, comprenant l'enregistrement de spectres d'absorption UV-Vis des GNR fonctionnalisées par du mannose ; enregistrés en particulier entre $\lambda$ = 400 à 1 000 nm, en outre particulièrement, lorsque la lecture de détection est sur la base de la région proche infrarouge comprise entre 700 et 900 nm.

**10.** Essai de liaison de MBL par LSPR selon l'une quelconque des revendications 8 à 9, comprenant la détermination d'un changement de position de pic de LSPR au fil du temps de contact avec l'échantillon de test.

**11.** Essai de liaison de MBL par LSPR selon la revendication 10, dans lequel le changement de position de pic de LSPR est exprimé par rapport à la position de pic au début de l'essai.

FIG.1

A.

B.

FIG. 2

FIG. 3

Man-PHPMA40 GNRs

Man-PHEA35 GNRs

FIG. 4

FIG. 5

FIG. 6

FIG. 7

FIG. 8

A.

B.

FIG. 9

A.

B.

FIG. 10

A.

B.

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EROL G** ; **SCHMIDT PP** ; **PANCARO A et al.** New nanostructures inhibiting human mannose binding lectin identified by a novel surface plasmon resonance assay. *Sensors Actuators B Chem*, 2022, vol. 360, 131661
- **PANCARO ALESSIA et al.** The polymeric glyco-linker controls the signal outputs for plasmonic gold nanorod biosensors due to biocorona formation. *NANOSCALE*, 24 June 2021, vol. 13 (24), 10837-10848
- **WANG Y. et al.** *Biosensors and Bioelectronics*, 15 November 2010, vol. 26 (3)
- **YOUNG JU SON et al.** *ACS Nano*, 26 November 2013, vol. 7 (11)
- **ZHAO Y** ; **TONG L** ; **LI Y et al.** Lactose-Functionalized Gold Nanorods for Sensitive and Rapid Serological Diagnosis of Cancer. *ACS Appl Mater Interfaces*, 2016, vol. 8 (9), 5813-5820
- **PANCARO A** ; **SZYMONIK M** ; **GEORGIOU PG et al.** The polymeric glyco-linker controls the signal outputs for plasmonic gold nanorod biosensors due to biocorona formation. *Nanoscale*, 2021, vol. 13, 10837-10848
- **BARRIENTOS AG** ; **DE LA FUENTE JM** ; **ROJAS TC** ; **FERNÁNDEZ A** ; **PENADÉS S**. Gold glyconanoparticles: Synthetic polyvalent ligands mimicking glycocalyx-like surfaces as tools for glycobiological studies. *Chem - A Eur J.*, 2003, vol. 9 (9), 1909-1921
- **RAJAGOPALAN R** ; **SALVI VP** ; **JENSENIUS JC** ; **RAWAL N.** New insights on the structural/functional properties of recombinant human mannan-binding lectin and its variants. *Immunol Lett.*, 2009, vol. 123 (2), 114-124
- **LEYVA A** ; **QUINTANA A** ; **SÁNCHEZ M** ; **RODRIGUEZ EN** ; **CREMATA J** ; **SÁNCHEZ JC**. Rapid and sensitive anthrone e sulfuric acid assay in microplate format to quantify carbohydrate in biopharmaceutical products : Method development and validation. *Biologicals*, 2008, vol. 36, 134-141

- **NIAZI A** ; **JORJANI O-N** ; **NIKBAKHT H** ; **GILL P**. A nanodiagnostic colorimetric assay for 18S rRNA of Leishmania pathogens using nucleic acid sequence-based amplification and gold nanorods. *Mol Diagn Ther.*, 2013, vol. 17 (6), 363-370
- **SABER R** ; **SHAKOORI Z** ; **SARKAR S** ; **TAVOOSIDANA G** ; **KHARRAZI S** ; **GILL P**. Spectroscopic and microscopic analyses of rod-shaped gold nanoparticles interacting with single-stranded DNA oligonucleotides. *IET nanobiotechnology*, 2013, vol. 7 (2), 42-49
- **ZHAO Y** ; **TONG L** ; **LI Y et al.** Lactose-Functionalized Gold Nanorods for Sensitive and Rapid Serological Diagnosis of Cancer. *ACS Appl Mater Interfaces.*, 2016, vol. 8 (9), 5813-5820
- **PITIROLLO O** ; **MICOLI F** ; **NECCHI F et al.** Gold nanoparticles morphology does not affect the multivalent presentation and antibody recognition of Group A Streptococcus synthetic oligorhamnans. *Bioorg Chem*, March 2020, vol. 99, 103815
- **HINTERWIRTH H** ; **KAPPEL S** ; **WAITZ T** ; **PROHASKA T** ; **LINDNER W** ; **LÄMMERHOFER M**. Quantifying thiol ligand density of self-assembled monolayers on gold nanoparticles by inductively coupled plasma-mass spectrometry. *ACS Nano.*, 2013, vol. 7 (2), 1129-1136
- **RETOUT M** ; **BRUNETTI E** ; **VALKENIER H** ; **BRUYLANTS G**. Limits of thiol chemistry revealed by quantitative analysis of mixed layers of thiolated-PEG ligands grafted onto gold nanoparticles. *J Colloid Interface Sci.*, 2019, vol. 557, 807-815
- **KAWASAKI T**. Structure and biology of mannan-binding protein, MBP, an important component of innate immunity. *Biochim Biophys Acta*, 1999, vol. 1473 (1), 186-195
- **KAWASAKI N** ; **KAWASAKI T** ; **YAMASHINA I**. Isolation and characterization of a mannan-binding protein from human serum. *J Biochem.*, 1983, vol. 94 (3), 937-947